# EUROPEAN PATENT APPLICATION

(11) **EP 1 656 933 A1**
(43) Date of publication of application: **17.05.2006**
(21) Application number: 04256997.0
(22) Date of filing: 11.11.2004
(51) Int. Cl.: A61K 31/198, A61P 1/16

(54) **A medicinal composition comprising L-alanine for non-alcoholic fatty liver disease and/or non-alcoholic steatohepatitis and use therof**

(71) Applicant: Ajinomoto Co., Inc., Tokyo 104-8315 (JP)
(72) Inventor: Takatsuki, Fumihiko, c/o Ajinomoto Co., Inc., Kawasaki-shi, Kanagawa 210-8681 (JP); Okano, Akira, c/o Ajinomoto Co., Inc., Kawasaki-shi, Kanagawa 210-8681 (JP)
(74) Representative: Denison, Christopher Marcus

(57) **Abstract**

The invention provides a medicinal composition comprising L-alanine as an active ingredient. This medicinal composition is safe and efficacious for prevention and/or therapeutic treatment of non-alcoholic fatty liver disease or non-alcoholic steatohepatitis patients. It provides a use of L-alanine therefor, and a method for preventing and/or treating non-alcoholic fatty liver disease or non-alcoholic steatohepatitis patients thereby.

## Description

The invention provides a medicinal composition comprising L-alanine as an active ingredient. This medicinal composition is safe and efficacious for prevention and/or therapeutic treatment of non-alcoholic fatty liver disease or non-alcoholic steatohepatitis patients. It is provides a use of L-alanine therefor, and a method for preventing and/or treating non-alcoholic fatty liver disease or non-alcoholic steatohepatitis patients thereby.

### [Background of the invention]

Non-alcoholic fatty liver disease / non-alcoholic steatohepatitis (henceforth "NASH") is a disorder characterized by hepatic steatosis, necro-inflammation and/or fibrosis that occurs in those who do not consume alcohol in amounts generally considered to be harmful to the liver (>20-30g/day). NASH is thought as a one of the features of "lifestyle-change-related metabolic syndrome" characterized by obesity, diabetes, insulin resistance etc. NASH can progress to severe liver failure including cirrohsis and is being recognized as a major cause of liver-related morbidity and mortality. Therefore, together with the recent increasing prevalence of those lifestyle-related-diseases, NASH is being increasing an important public health problem. Some pharmacologic agents have been trialed. At this time, however, no specific medicine and treatment is established based on the evidence about their safety and efficacy in the management of NASH (non-patenting reference 1).
Nutritional modification is one of the attractive therapies in chronic disease such as NASH, since these approaches are relatively safe and tolerable in general. Although there are many reports of nutritional effects of amino acids, there are not so many reports of the pharmacological effects of amino acids that are relevant to liver failure.
The amino acid L-alanine has been studied in vivo by using several animal models of acute hepatic injury induced by the administration of D-galactosamine or carbontetrachloride (non-patenting reference 2 and 3). In the studies using primary hepatocytes injured by D-galactosamine, L-alanine also exhibited hepatoprotective effect in dose-dependent manner through being metabolized in the TCA cycle in vitro (non-patenting reference 4). Patent references 1, 2, and 3 disclose pharmacological efficacy of L-alanine for alcoholic liver failure, viral hepatitis, or liver regeneration based on animal data (). Although there are limited data from human studies, L-alanine has been experimentally used in a patient with an advanced stage of primary biliary cirrhosis (PBC) for 8 weeks and has shown positive therapeutic effects (patent reference 4). However, there is no report of safe and efficacious long-term treatment of L-alanine for patients with chronic, metabolic liver disease such as NASH.
Therefore, development of a safe and effective new medicinal composition and a method for prevention and/or therapeutic treatment of NASH patients thereby are highly desired.

[Patent reference 1] JP 5-213746 A
[Patent reference 2] JP 5-221858 A
[Patent reference 3] JP 5-229940 A
[Patent reference 4] JP 11-853914 A
[Non-patenting reference 1] Gastroenterology, 123: 1702-1704,1705-1725 (2002)
[Non-patenting reference 2] Hepatology 24: 185-191 (1996)
[Non-patenting reference 3] Hepatology 24: 1211-1216 (1996)
[Non-patenting reference 4] Biochem. Biophys. Res. Commun. 291: 738-743 (2002)

### [Detailed Description of the invention]

The present inventors produced the medicinal composition containing L-alanine, and found out it could serve for the prevention or therapeutic treatment of NASH patients. That is, one embodiment of this invention is the medicinal composition containing amino acids, especially L-alanine as the ingredient, for prevention and/or therapeutic treatment of NASH.
Another embodiment of the invention is the use of L-alanine therefor.
Another embodiment of the invention is the method for treatment of NASH patients thereby.

L-alanine used as the active ingredient in the present invention includes L-alanine itself, pharmaceutically acceptable salts of L-alanine, or the amino acid derivatives that are metabolized to L-alanine and exhibit the aforementioned pharmaceutical effects, for example alanyl-glutamine. Acids capable of forming pharmaceutically acceptable salts with L-alanine include inorganic acids such as hydrogen chloride, hydrogen bromide, sulfuric acid and phosphoric acid, and organic acids such as acetic acid, lactic acid, citric acid, tartaric acid, maleic acid, fumaric acid and monomethylsulfuric acid, for example. Bases capable of forming pharmaceutically acceptable salts with L-alanine include inorganic bases such as sodium, potassium, calcium and ammonia, and organic bases such as ethylenediamine, propylenediamine, ethanolamine, monoalkylethanolamines, dialkylethanolamines, diethanolamine and triethanolamine, for example.

In the present invention, L-alanine may be used together with other one or more pharmaceutical components (pharmaceutically active substances), for example, mixed with or in combination with hepatoprotectants, anti-diabetics, anti-lipidemics, anti-hypertensives, anti-obesities, anti-oxidants and/or anti-inflammatory agents. Hepatoprotectants include, for example, ursodeoxycholic acid or betaine. Anti-diabetics include, for example, insulin, an insulin derivative, sulfonylureas (such as tolbutamide, gllclazide, glibenclamide or glimepiride), first-acting insulin secretagogues (such as nateglinide, repaglinide or mitiglinide), alpha-glucosidase inhibitors (such as acarbose, voglibose or miglitol), biguanides (such as metformin or phenformin) or thiazolidinediones or equivalent PPAR-gamma modulators (such as pioglitazone, rosiglitazone or troglitazone). Anti-lipidemics include, for example, HMG-CoA reductase inhibitors (such as lovastatin, pravastatin, simvastatin, fluvastatin, cerivastatin or atorvastatin) or fibric acids (such as clofibrate, fenofibrate or gemifibrozil. Anti-hypertensives Include, for example, alpha adrenergic blockers, beta blockers, calcium channel blockers (dihydropyridines or non-dihydropyridines), ACE inhibitors or angiotensin II receptor antagonists. Anti-obesities include, for example, centrally acting adrenergic agents (such as phentermine), centrally acting serotonin/NE agents (such as sibutramine) or lipase inhibitor (such as orlistat). Anti-oxidants include, for example, vitamins (such as vitamin C or vitamin E), N-acetyl cystein, probucol, eicosapentaic acid and esters thereof. Anti-inflammatory agents include, for example, cytokine inhibitors (such as pentoxifylline). Further, such other pharmaceutical components may be in the salt forms or the derivatives thereof, and also in the salt forms or the compounded forms with the above essential and effective components as objective in the present invention provided they exhibit the pharmacological activity required in the present invention.

The quantity of L-alanine, in a medicinal composition containing other pharmaceutical components such as hepatoprotectants, anti-diabetics, anti-lipidemics, anti-hypertensives, anti-obesities, anti-oxidants and/or anti-inflammatory agents, is not particularly limited. The content of L-alanine may be from 0.1 to 99.9 mass % (of the total composition), such that the other ingredients combined total 99.9 to 0.1 mass %.

The medicinal composition for NASH in the present invention can be given by, for example, oral, intravenous, subcutaneous or intra-muscular administration. It is usable in a dosage form suitable for the administration method, such as a form for the internal administration, e. g. a powder, granules, dry syrup (to be taken after suspending it in a suitable amount of water or to be taken as it is like the powder or granules), tablets (including chewable tablets), capsules and a liquid for internal use; or a form for the intravenous injection. Such a preparation can be prepared by using a variety of pharmacologically acceptable pharmaceutical assistant substances for an ordinary preparation by an ordinary method. Such substances may be properly selected according to the form of the pharmaceutical preparations, including one or more of, for example, diluents, binders, coating agents, bases, suspending agents, corrigents, flavors, lubricants and the like. Examples of such substances include inert diluents (such as lactose, calcium carbonate and calcium phosphate), binders (such as gum arabic, corn starch, gelatin, hydroxypropylcellulose and polyvinylpyrrolidone), coating agents (such as hydroxypropylcellulose), bases (such as mannitol; excipients such as alginic acid, corn starch and pregelatinized starch), suspending agents (such as crystalline cellulose carmellose sodium), corrigents (such as sucrose, lactose, aspartame and saccharin), flavors (such as peppermint, menthol, lemon oil and cherry) and lubricants (such as magnesium stearate and talc).

As for the dosage of the medicinal composition for NASH in the present invention, the dose of the active ingredient for adults is at least 1 g, preferably 5 to 50 g and more preferably 10 to 20 g a day. In a severe case, it may be increased and in more mild cases the dosage may be decreased. With respect to frequency and timing of doses, once dose per several days or once a day may also be acceptable. Usually, however, it will be administered several times a day, for example, in from 2 to 4 divided doses.

In the case that L-alanine is mixed with or in combination with other pharmaceutical components such as hepatoprotectants, anti-dlabetics, anti-lipidemics, anti-hypertensives, anti-obesities, anti-oxidants and/or anti-inflammatory agents, the amount employed of these components is that amount suitable as an effective quantity of each medicine which is already developed as a medicine.

### [Examples]

The following examples will further illustrate the present invention, which are provided herein for purposes of illustration only, and are not intended to be limiting unless otherwise specified.

### [Example.1] Manufacture of the medicinal composition containing L-alanine

Six gram of powdered L-alanine was filled into aluminum-laminate bags and sealed.
This package was provided as the medicinal composition containing L-alanine for medication.

### [Example.2] Treatment of patients with NASH by medicinal composition containing L-alanine

Patients diagnosed as NASH take the medicinal composition produced in example 1 once a day. According to the patient's situation, the amount of medication is increased twice a day at 5th week and three times a day at 9th week. Study design such as patient population, inclusion and exclusion criteria, laboratory investigation, histological evaluation and statistic analysis are based on the standard method (Am.J.Gastroenterol.2001; 96: 2711-2717); monitoring laboratory tests including liver biochemistries (ALT, AST) are performed during the study. Histological evaluation such as liver biopsy is performed at entry and repeated at the termination of medication.
A significant improvement in serum levels of ALT and/or AST occurs during treatment. It is related to a marked improvement in the histological score assessed by the steatosis and necro-inflammatory grading and fibrosis staging according to the method of Brunt. Therefore, the medicinal composition containing L-alanine in this invention is useful for NASH patients.

### [Example.3]

The effects of L-alanine for NASH was examined in choline-deficient diet (hereinafter CDD) rat model (Biochem.Biophys.Res.Commun., 315, 187-195 (2004)) ,which shows NASH-like pathlogical change in the liver.
Male Wistar rats (7 weeks of age, 5 rats per group) received CDD (Dyets Co.) for 4 weeks. All rats fed CDD increased liver weight (14.5 to 20.0 g), and showed macrovesicular hepatic steatosis in histological examination.
Ursodeoxycholic acid (UDCA) is a drug which showed hepatoprotective effect for patients with primary biliary cirrhosis and has recently trialed in the treatment of NASH.
However, UDCA did not improve NASH-like pathological change in CDD rat model; all of 5 rats received CDD mixed with 0.25% UDCA for 4 weeks showed increase of liver weight and macrovesicular hepatic steatosis.
On the other hand, in the group of 5 rats received CDD mixed with 4% L-alanine for 4 weeks, one rat is normalized in liver weight (10.6g). Histological fat accumulation was not recognized in the liver in this rat.

### [Example.4]

It has been supposed that the increase of the amount of fat intake in the last few decades is one of the causes of the recent increase of the number of patients with NASH.
Therefore, the effect of L-alanine on the hepatic triglyceride contents in the high-fat diet (hereinafter HFD)-induced fatty liver rat model.
Male GK rats (9 weeks of age, 6 rats per group) received HFD (containing 30% beef tallow) for 6 weeks. Triglyceride contents in the liver tissue was increased to 29.1 to 49.3 mg per gram of liver tissue, compared with those (14.1 to 24.2 mg) in the group fed with standard diet (containing 5% soy-been oil; hereinafter SD).
Pioglitazone is a drug, which acts as an insulin-sensitizer for the patients with diabetes. Recent clinical pilot studies raise the possibility of clinical utility of pioglitazone for NASH.
However, pioglitazone did not improve fatty liver in HFD-induced fatty liver rat model; all of 6 rats received HFD mixed with 0.01% pioglitazone for 6 weeks decreased hepatic triglyceride contents slightly (26.7 to 48.4 mg per 1g of liver tissue), but no individual showed the significant decrease of hepatic triglyceride contents to the range in SD group.
On the other hand, in the group of 6 rats received CDD mixed with 4% L-alanine for 6 weeks, one rat is reduced in the liver triglyceride contents (19.6 mg) in the level of SD group.
In addition, weight gain was reported as the main side effect in a pilot clinical study of piogritazone for NASH. In this HFD-induced fatty liver rat model, body weight of the rats fed with HFD + pioglitazone for 6 weeks was significantly more increased, compared with those in the group fed with HFD alone. On the other hand, such an increase in weight was not observed in L-alanine treated rats.

| Group | Body Weight (g) | |
|---|---|---|
| | Pre | Post (at 6 weeks) |
| SD | 220.7±16.2 | 317.3±11.7 |
| HFD | 217.5±11.4 | 331.0± 8.6 |
| HFD+L-alanine | 219.8±13.0 | 331.8±20.5 |
| HFD+pioglitazone | 222.1± 9.9 | 367.0±19.3 |

| | | |
|---|---|---|
| Data represent means ± SD (n=6) | | |

## Claims

1. Use of L-alanine in the preparation of a medicament for the prevention or treatment of non-alcoholic fatty liver disease and/or non-atcaholic steatohepatitis.

2. A medicinal composition comprising L-alanine as an active ingredient for the prevention and/or therapeutic treatment of non-alcoholic fatty liver disease and/or non-alcoholic steatohepatitis.
